Europäisches Patentamt

European Patent Office

Office européen des brevets

(11) Veröffentlichungsnummer: **0 388 822**
**A2**

(12) **EUROPÄISCHE PATENTANMELDUNG**

(21) Anmeldenummer: **90105049.2**

(22) Anmeldetag: **17.03.90**

(51) Int. Cl.5: **A61M 3/02**

(30) Priorität: **21.03.89 DE 8903555 U**

(43) Veröffentlichungstag der Anmeldung:
**26.09.90 Patentblatt 90/39**

(84) Benannte Vertragsstaaten:
**AT CH DE FR GB IT LI NL SE**

(71) Anmelder: **Mendel, Volker, Dr.**
**Am Hollerbusch 11**
**D-3000 Hannover 51(DE)**

(72) Erfinder: **Mendel, Volker, Dr.**
**Am Hollerbusch 11**
**D-3000 Hannover 51(DE)**
Erfinder: **Haindl, Hans-Günter**
**Schöne Aussicht 4**
**D-3508 Melsungen(DE)**
Erfinder: **Brethauer, Ulrich**
**Rhönstrasse 2**
**D-3501 Körle(DE)**

(74) Vertreter: **Leine, Sigurd, Dipl.-Ing. et al**
**Dipl.-Ing. Sigurd Leine Dipl.-Phys. Dr.**
**Norbert König Patentanwälte**
**Burckhardtstrasse 1**
**D-3000 Hannover 1(DE)**

(54) **Auffangbeutel mit Anschlussteil.**

(57) Auffangbeutel mit Anschlußteil für ein Set zur intraoperativen Darmspülung mittels eines großlumigen Überleitungsgerätes, an das über einen Zuführungsschlauch eine Verweilkanüle angeschlossen ist. Anschlußteil und Auffangbeutel sind als extrudierter gerader Folienschlauch aus hochfester PVC ausgebildet, dessen eines Ende versiegelt und dessen anderes Ende einen eingeschweißten Stutzen aufweist, in dem ein Verschlußstopfen steckt. Der Auffangbeutel ist preiswert herstellbar sowie bei der Anwendung und Entsorgung zuverlässig und hygienisch.

FIG.1

## Auffangbeutel mit Anschlußteil

Die Erfindung bezieht sich auf einen Auffangbeutel mit Anschlußteil für ein Set zur intraoperativen Darmspülung mittels eines großlumigen Überleitungsgerätes, an das über einen Zuführungsschlauch eine Verweilkanüle angeschlossen ist.

Bei stenosierenden Prozessen des Dickdarmes kann vor der Operation häufig keine zufriedenstellende Reinigung des proximalen Darmabschnitts erreicht werden: diese ist aber nötig, um erfolgversprechend eine primäre Anastomosierung des Darmes vornehmen zu können. In diesen Fällen wird zumeist ein proximaler Entlastungs-Anus praeter gelegt, der nach dem Verheilen der Anostomose dann wieder verschlossen und zurückverlegt wird. Diese Prozedur ist für den Patienten körperlich und psychisch belastend.

Zur Vermeidung dieser für den Patienten belastenden Methodix wurde von H.A.F. Dudley die intraoperative Darmlavage empfohlen, bei der ein Set benutzt wird, das aus einem dicken langen Kunststoff-Wellrohr, einem großen Kunststoff-Entsorgungssack und einem Foley- Katheter besteht, der an ein Überleitungsgerät mit Spüllösung angeschlossen ist (Br.J. Surg. Vol. 67 (1980) 80-81. Das Kunststoff-Wellrohr wird mit einem Ende in das proximale Darmende vorgeschoben und mit zwei Nabelschnurbändern fest eingeknotet. Der Foley-Katheter wird in das Zökum vorgeschoben eingeknotet und geblockt. Das andere Ende des Kunststoff-Wellrohres ragt in den Kunststoff-Entsorgungssack hinein, der sich neben dem Operationstisch befindet. Die Öffnung des Entsorgungssackes wird auf das Ende des Wellrohres gebunden. Auch zur Entsorgung des gefüllten Entsorgungssackes wird dessen Öffnung lediglich zugeschnürt. Eine besonders kritische Stelle dieses Sets ist die Verbindung zwischen Wellrohr und Entsorgungssack und der Verschluß des gefüllten Entsorgungssakkes. Ein versehentliches Trennen der Verbindung oder Öffnen des gefüllten Entsorgungssackes hat katastrophale Konsequenzen, die Chirurgen von dem Einsatz eines solchen improvisierten Abfluß- und Auffangsystems abhalten.

Bei einem anderen bekannten Set (Br.J. Surg. 1985, Vol. 72, September, 708-711) ist das Kunststoff-Wellrohr mit separatem Auffangbeutel ersetzt worden durch ein einstückiges Schlauch-Beutelsystem aus Polyethylen. Ein langer flacher Schlauch dient dabei als Anschlußteil zur Verbindung der Darmöffnung mit dem Auffangbeutel, der unsymmetrisch an das äußere Ende des Schlauches angeformt ist. Zur Herstellung des Schlauches mit Auffangbeutel sind zwei Folienzuschnitte erforderlich, die jeweils aus einem geräden Streifen bestehen, der an einem Ende einen großen seitlichen Lappen aufweist. Die beiden aufeinanderliegenden Folienzuschnitte müssen ringsum zusammengeschweißt werden. Da der Beutel ein Fassungsvermögen von mehr als fünf Litern haben muß, sind die Zuschnitte groß und zum Verschweißen werden Spezial-Schweißformen beträchtlicher Abmessungen benötigt, die die Herstellung solcher Auffangbeutel mit angeformtem Anschlußteil zusätzlich verteuern. Außerdem ist nachteilig, daß Polyethylen-Folie verwendet wird, da diese nicht reißfest ist und der unter dem Operationstisch liegende Auffangbeutel bei Anstoß platzen kann. Ferner neigt die Polyethylen-Folie zum Kleben, so daß ein freier offener Innenquerschnitt des schlauchartigen Anschlußteiles beim Einlaufen der Spülflüssigkeit nicht gewährleistet ist. Jede Drosselung des Abflusses der Spülflüssigkeit behindert jedoch den Durchspülungsvorgang und stellt den Reinigungseffekt mit der vorgegebenen Spüllösungsmenge infrage. Da das Fassungsvermögen des Überleitungsgerätes auf das Fassungsvermögen des Auffangbeutels abgestimmt ist, wäre bei Notwendigkeit einer zusätzlichen Spülflüssigkeitsmenge das Auswechseln des Auffangsystems erforderlich, wodurch sich eine zusätzliche Belastung des Patienten und des Chirurgen ergeben würde.

Das freie Ende des Schlauches wird in den Darm eingebunden. Zum Verschluß des gefüllten Auffangsystems wird das Schlauchende zugeklemmt. Die Zuverlässigkeit solcher Maßnahmen ist fraglich.

Der Erfindung liegt die Aufgabe zugrunde, ein Set zur intraoperativen Darmspülung dadurch zu verbessern, daß das Auffangsystem preiswert herstellbar sowie bei der Anwendung und Entsorgung zuverlässiger und hygienischer ist.

Diese Aufgabe wird erfindungsgemäß dadurch gelöst, daß Anschlußteil und Auffangbeutel als extrudierter gerader Folienschlauch aus hochfestem PVC ausgebildet sind, dessen eines Ende versiegelt ist und dessen anderes Ende einen eingeschweißten Stutzen aufweist, in dem ein Verschlußstopfen steckt.

Die Herstellung von Anschlußteil und Auffangbeutel als schlauchextrudierte Folie ohne jegliche Erweiterungen oder Ausbauchungen ermöglicht eine preiswerte Herstellung des Auffangsystems, weil weder Folienzuschnitte noch Schweißapparaturen zur Randverschweißung benötigt werden. Der umfangsmäßig geschlossene gerade Folienschlauch kann zur Erzielung des gewünschten Fassungsvermögens von einem Ende zum anderen eine Länge von ca. 2 m und einen gleichbleibenden Durchmesser von ca. 12 cm haben. Da er aus hochfestem PVC gefertigt ist, ist er so stabil, daß er selbst bei versehentlichem Drauftreten nicht beschädigt wird.

Über den Stutzen wird das proximale Darmende gezogen und der Darm wird durch Umschlingungsligatur mit kräftiger Naht auf dem Stutzen gesichert. Diese Verbindung ist zuverlässig haltbar und während des Spülvorganges dicht. Die Festlegung des Darmes auf den Stutzen wird dadurch abrutschsicherer, daß der äußere Rand des Stutzens von einer Ringwulst umgeben ist. Die Spülung wird solange fortgesetzt, bis die Flüssigkeit in dem Folienschlauch klar ist. Sodann wird der proximale Dickdarmstumpf oberhalb des Stutzenanschlusses mit einer weichen Darmklemme abgeklemmt und das auf den Stutzen gestülpte Darmende wird abgesetzt. Dann wird der Stutzen mit dem Verschlußstopfen verschlossen und der Folienschlauch kann problemlos entsorgt werden. Die Darmstümpfe werden anschließend primär anastomosiert, ohne daß ein Entlastungs-Anus praeter gelegt werden muß.

In vorteilhafter Ausgestaltung der Erfindung ist der Folienschlauch auf der Innenfläche aufgerauht. Die Mikrorauhigkeit verhindert, daß die einander berührenden Flächen des flachliegenden Folienschlauches zusammenkleben. Die Kapillarkräfte der einströmenden Flüssigkeit öffnen verzögerungslos den gesamten Folienschlauchquerschnitt, so daß durch behinderungsfreies Abfließen der am Appendix durch die Verweilkanüle eingeführten Spülflüssigkeit eine gründliche Ausspülung des Dickdarminhaltes in den Folienschlauch gewährleistet ist.

In vorteilhafter Ausgestaltung der Erfindung weist der Verschlußstopfen einen langen konischen Becherschaft mit geschlossenem Boden auf, an dessen breitem offenen Ende sich ein Griffring mit Rändelung befindet. Der Becherschaft läßt sich bis zur Anlage des unteren Randes des Griffringes gegen die Stirnfläche der Ringwulst des Stutzens in den Stutzen des Folienschlauches hineinschieben und dichtet den Verschluß zuverlässig ab. In der Außenfläche des Becherschaftes sind Längsrillen ausgebildet, die an seinem geschlossenen schmalen Ende offen sind und die am anderen Ende mit Abstand vor dem Griffring enden. Diese Längsrillen dienen als Luftkanäle, durch die in dem Folienschlauch gefangene Luft beim Einschieben des Verschlußstopfens in den Stutzen entweichen kann, so daß der verschlußstopfen behinderungsfrei in seine abdichtende bis zum Anschlag eingesteckte Endposition vorgeschoben werden kann. Sie gewährleisten außerdem bei der Sterilisation, die bei halb eingeschobenem Stopfen erfolgt, den sicheren Ein- und Austritt des Sterilisationsgases. Da die Länge des Becherschaftes und des Stutzens einander angepaßt sind, ist ein zuverlässiger Verschluß gewährleistet. Der hohle Becherschaft verbilligt die Herstellung des Verschlußstopfens durch geringen Materialverbrauch.

In der Zeichnung ist ein Ausführungsbeispiel der Erfindung schematisch dargestellt.

Es zeigen:

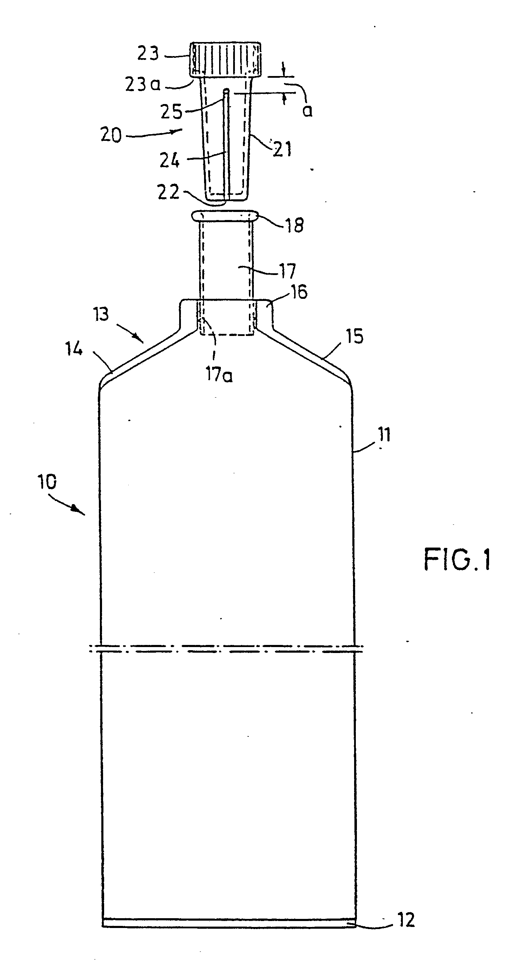

Fig. 1 eine Draufsicht auf das flachliegende Auffang system mit herausgezogenem Verschlußstopfen, und

Fig. 2 das Anwendungsschema des Sets zur intraoperativen Darmspülung.

Ein Auffangbeutel 10 mit Anschlußteil besteht erfindungsgemäß aus einem extrudierten geraden Folienschlauch 11 aus hochfester PVC-Folie, die innen aufgerauht ist, um ein Kleben sicher zu verhindern. Der Folienschlauch 11 ist ringsum nahtlos geschlossen und erstreckt sich ohne Ausbauchung mit einer gleichmäßigen Breite von ca. 12 cm über eine Länge von etwa 2 m. Es ergibt sich ein Fassungsvermögen von mindestens 5 Litern Spüllösung mit Darminhalt. Während des Spülvorganges liegt die Hauptlänge des Folienschlauches 11 unter dem Operationstisch. Sein eines Ende 12 ist durch eine quergerichtete Siegelnaht verschlossen und sein anderes Ende 13 ist durch zwei seitliche Siegelnähte 14,15 verjüngt und mit einer angeformten kreisringförmigen Tülle 16 versehen, die koaxial angeordnet ist. Das PVC-Material ist durchsichtig. In die Tülle 16 ist das Ende 17a eines innen und außen kreiszylindrischen Stutzens 17 eingeschweißt, der eine gewisse radiale Nachgiebigkeit hat. Der längere Teil des Stutzens 17 steht über die Tülle 16 nach außen vor. Der äußere Rand des Stutzens 17 ist von einer Ringwulst 18 umgeben, die im Querschnitt halbkreisförmig gerundet ist.

Zum Verschluß des Stutzens dient ein Verschlußstopfen 20 aus Kunststoff. Der Verschlußstopfen 20 ist hohl. Er besteht aus einem langen konischen Becherschaft 21, der durch einen boden 22 an seinem schmaleren Ende verschlossen ist und der an seinem breiteren offenen Ende einen Griffring 23 trägt. Der Griffring 23 ist auf der Außenfläche gerändelt. Auf der Außenfläche des Becherschaftes 21 befinden sich mehrere Längsrillen 24, die an der Bodenseite des Verschlußstopfens 20 offen sind und bei 25 mit Abstand zu dem Griffring 23 geschlossen sind. Auf diese Weise wird beim Einschieben des Verschlußstopfens 20 ein Auslaß von in dem Folienschlauch 11 gefangenen Gasen durch die Längsrillen 24 ermöglicht, der eine vorzeitige Blockierung des Vorschubes des Verschlußstopfens 20 verhindert. Bei bis zum Anschlag tief in den Stutzen 17 eingestecktem Verschlußstopfen 20 liegt das Becherschaftstück a abdichtend gegen die Innenfläche des Stutzens 17 an. Zusätzliche Abdichtung bietet die Anpressung der Fläche 23a des Griffringes gegen die Stirnfläche der Ringwulst 18.

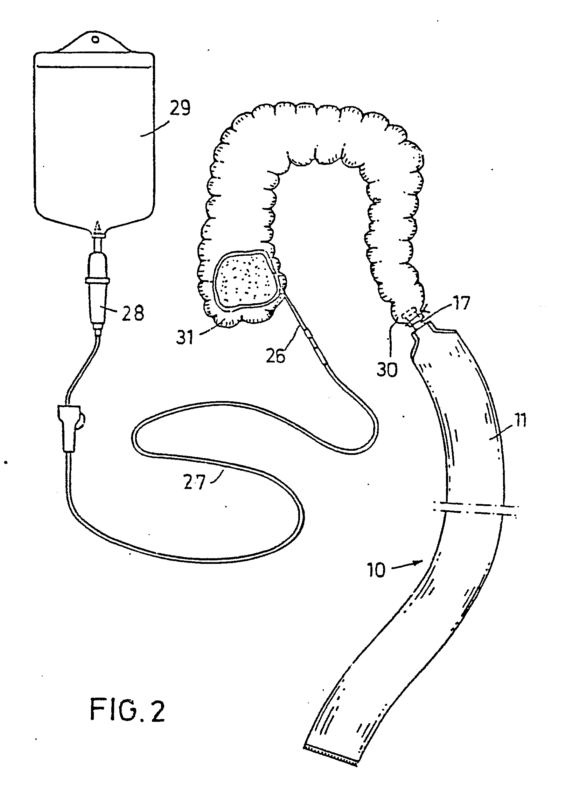

Das operative Vorgehen wird anhand der Darstellung nach Fig. 2 erläutert. Zur Spülung des proximalen Dickdarmanteiles bei der Operation stenosierender Dickdarmprozesse am linksseitigen Kolon wird nach Resektion des stenosierenden Prozesses der proximale Dickdarmstumpf 30 über den Stutzen 17 gezogen und auf diesem durch Umschlingungsligatur mit kräftiger Naht gesichert. Sodann wird am Appendix 31 eine Verweilkanüle 26 in den Darm eingeführt und durch Umstechungsligatur fixiert. Mit der Verweilkanüle 26 wird ein Zuführungsschlauch 27 konnektiert, der über eine Tropfkammer 28 an einen Spülflüssigkeitsbeutel 29 angeschlossen ist. Der Spülflüssigkeitsbeutel 29 enthält eine geeignete, auf Körpertemperatur vorgewärmte Spüllösung. Ausdem Überleitungsset fließen in kürzester Zeit 3 bis 4 Liter Spüllösung in den Dickdarm ein, die den Dickdarminhalt durch den Stutzen 17 in den Folienschlauch 11 spülen. Wenn die Spülflüssigkeit klar ist, wird nach Herausziehen der Verweilkanüle 26 aus dem Darm und Verschluß des Punktionsloches das auf den Stutzen 17 gestülpte Darmende 30 abgesetzt. Der Stutzen 17 wird mit dem Verschlußstopfen 20 verschlossen und die Darmstümpfe werden primär anastomosiert. Der verschlossene gefüllte Folienschlauch 11 kann problemlos entsorgt werden. Durch seine Herstellung aus hochfestem PVC und das Fehlen von Längs-Schweißnähten ist er so stabil, daß er weder bei der Anwendung durch versehentliches Drauftreten noch im Entsorgungszustand beschädigt werden kann.

**Ansprüche**

1. Auffangbeutel mit Anschlußteil für ein Set zur intraoperativen Darmspülung mittels eines großlumigen Überleitungsgerätes, an das über einen Zuführungsschlauch eine Verweilkanüle angeschlossen ist,
**dadurch gekennzeichnet,**
daß Anschlußteil und Auffangbeutel als extrudierter gerader Folienschlauch (11) aus hochfestem PVC ausgebildet sind, dessen eines Ende (12) versiegelt ist und dessen anderes Ende (13) einen eingeschweißten Stutzen (17) aufweist, in dem ein Verschlußstopfen (20) steckt.

2. Auffangbeutel nach Anspruch 1, dadurch gekennzeichnet, daß der äußere Rand des Stutzens (17) von einer Ringwulst (18) umgeben ist.

3. Auffangbeutel nach Anspruch 1 oder 2, dadurch gekennzeichnet, daß der Folienschlauch (11) auf der Innenfläche aufgerauht ist.

4. Auffangbeutel nach einem der Ansprüche 1-3, dadurch gekennzeichnet, daß der Verschlußstopfen (20) einen langen konischen Becherschaft (21) mit geschlossenem Boden (22) aufweist, an dessen breitem offenen Ende sich ein Griffring (23) mit Rändelung befindet.

5. Auffangbeutel nach Anspruch 4, dadurch gekennzeichnet, daß in der Außenfläche des Becherschaftes (21) Längsrillen (24) ausgebildet sind, die am geschlossenen schmalen Ende des Becherschaftes (21) offen sind und die am anderen Ende mit Abstand vor dem Griffring (23) enden.

6. Auffangbeutel nach Anspruch 4 oder 5, dadurch gekennzeichnet, daß die Länge des Becherschaftes (21) und des Stutzens (17) einander angepaßt sind.

7. Auffangbeutel nach einem der Ansprüche 1-6, dadurch gekennzeichnet, daß der Folienschlauch (11) etwa 200 cm lang und über seine ganze Länge etwa 12 cm breit ist.

FIG.1

FIG.2